# EUROPÄISCHE PATENTSCHRIFT

(11) **EP 2 266 544 B1**
(45) Veröffentlichungstag und Bekanntmachung des Hinweises auf die Patenterteilung: **28.06.2017**
(21) Anmeldenummer: 10012394.2
(22) Anmeldetag: 21.08.2006
(51) Int. Cl.: A61K 9/10, A61K 9/50, A61K 9/51, A61K 33/00, A61K 33/24, A61K 33/26, A61K 33/30, A61K 33/32, A61K 33/34, A61K 31/136, A61K 31/282, A61K 31/407, A61K 31/513, A61K 31/546, A61K 41/00, A61K 45/06, A61N 1/40, B82Y 5/00

(54) **VERFAHREN ZUR EINSCHLEUSUNG VON THERAPEUTISCHEN SUBSTANZEN IN ZELLEN**
METHOD FOR INTRODUCING THERAPEUTIC SUBSTANCES INTO CELLS
PROCEDÉ D'INTRODUCTION DE SUBSTANCES THERAPEUTIQUES DANS DES CELLULES

(30) Priorität: 19.08.2005 DE 102005039579; 26.08.2005 US 711407 P
(43) Veröffentlichungstag der Anmeldung: 29.12.2010
(62) Teilanmeldung aus: 06775877.1
(73) Patentinhaber: MagForce AG, 12489 Berlin (DE)
(72) Erfinder: Jordan, Andreas, 14513 Teltow (DE); Waldöfner, Norbert, 41767 Duisburg (DE); Scholz, Regina, 37441 Bad Sachsa (DE)
(74) Vertreter: Bösl, Raphael Konrad

(56) Entgegenhaltungen:
- WO-A1-2006/089290
- DE-A1- 10 020 376
- DE-A1- 19 726 282

## Beschreibung

Die vorliegende Erfindung betrifft Zusammensetzungen enthaltend Nanopartikel und Verwendungen dieser Zusammensetzung zur Einschleusung von therapeutisch wirksamen Substanzen in Zellen, insbesondere Krebszellen. Die Partikel sind chemisch so gestaltet, dass eine hohe intrazelluläre Aufnahme in die Zellen stattfindet. Für die Einschleusung in die Zellen muss keine direkte Bindung zwischen Nanopartikel und der therapeutisch wirksamen Substanz vorliegen. Die pharmazeutischen Zusammensetzungen aus Nanopartikel und Antikrebsmittel führen zu einer gesteigerten Wirkung des Antikrebsmittels und zu verringerten Nebenwirkungen.

Es ist bekannt, dass Nanopartikel von Zellen (insbesondere Tumorzellen) durch Endozytose aufgenommen werden können. Ein Verfahren zur Herstellung von zellgängigen Nanopartikeln ist in DE 197 26 282.1 genannt. Die Aufnahme der Nanopartikel kann durch in-vitro Untersuchungen an hochreinem Zellmaterial untersucht werden. In der DE 199 12 798 C1 sind Methoden genannt, mit deren Hilfe man beliebige Zellen aus Gewebematerial in Kultur bringen kann. Durch diese Methoden ist es möglich, die Partikel chemisch so zu gestalten, dass eine hohe Aufnahme in bestimmte Tumorzellen stattfindet. Methoden zur Wirksamkeitssteigerung von therapeutischen Substanzen durch Ankopplung an Nanopartikel als Trägersysteme sind ebenfalls bekannt und auch Gegenstand der Forschung. So wird in DE 10059151 A eine Ankopplung der Substanzen durch ionische Wechselwirkung verfolgt, wobei das Konjugat im Tumorgewebe angereichert werden soll. Die Freisetzung der therapeutischen Substanz erfolgt dann allerdings im Interstitium und nicht intrazellulär. Die Einschleusung von Nanopartikeln in Tumorzellen mit Hilfe von Antikörpern oder Peptiden (z.B. TAT-Peptide) ist ebenfalls bekannt. Diese Art der Einschleusung führt allerdings nur zu einer vergleichsweise geringen Anreicherung von Nanopartikeln in Tumorzellen und kann daher nicht für therapeutische Zwecke genutzt werden.

Aufgabe der vorliegenden Erfindung ist es, Zusammensetzungen und Verwendungen dieser Zusammensetzungen zur Behandlung und Prophylaxe von Krebserkrankungen bereitzustellen.

Die Aufgabe wird durch die Nanopartikel in der Verwendung gemäß Anspruch 1 gelöst. Weitere vorteilhafte Ausgestaltungen ergeben sich aus den abhängigen Ansprüchen, den Beispielen sowie Figuren und der Beschreibung. Beschrieben werden pharmazeutische Zusammensetzungen, welche aus Nanopartikeln mit einer hohen Affinität zu entarteten Zellen, mindestens eine therapeutisch wirksame Substanz, insbesondere ein Antikrebsmittel und mindestens einem pharmakologisch verträglichen Träger, Hilfsstoff und/oder Lösungsmittel bestehen.

Als pharmakologisch verträgliche Träger, Hilfsstoffe und/oder Lösungsmittel können die in der Galenik (Pharmazeutische Technologie) üblichen Stoffe eingesetzt werden, wobei flüssige pharmazeutische Zusammensetzungen bevorzugt sind.

Als Lösungsmittel eignen sich Wasser oder physiologische Kochsalzlösung. Falls erforderlich, können auch Cosolventien wie beispielsweise Ethanol bis vorzugsweise zu einer Menge von 10 Vol.-% eingesetzt werden.

Bei diesen pharmazeutischen Zusammensetzungen handelt es sich insbesondere um Infusions- oder Injektionsiösungen. Derartige Lösungen der Nanopartikel in beispielsweise physiologischer Kochsalzlösung sind für die interstitielle bzw. intratumorale Applikation geeignet. Eine intraarterielle oder intravenöse Applikation ermöglicht ferner eine systemische, den ganzen Körper betreffende Therapiemöglichkeit für nicht solide und/oder metastasenbildende Tumorarten.

Die Nanopartikel oder Nanoteilchen und die mindestens eine therapeutische Substanz, insbesondere das mindestens eine Antikrebsmittel, können in einer einzigen vorzugsweise Injektionslösung oder Infusionslösung enthalten sein. Die pharmazeutische Zusammensetzung kann aber auch aus zwei Lösungen bestehen, wobei die eine die Nanopartikel und die andere die mindestens eine therapeutisch wirksame Substanz, insbesondere das mindestens eine Antikrebsmittel enthält, und beide Lösungen gleichzeitig oder zeitgleich appliziert werden. Gemäß der vorliegenden Erfindung werden die Nanopartikel zusammen mit einem Antikrebsmittel verabreicht, so dass die Nanopartikel und das Antikrebsmittel gleichzeitig an der entarteten Zelle anwesend sind, wobei die Nanopartikel und das Antikrebsmittel in getrennten Lösungen verabreicht werden.

Erfindungswesentlich ist, dass sich überraschend herausgestellt nat, dass die hierin beschriebenen Nanopartikel die Fähigkeit besitzen, therapeutische Substanzen, insbesondere Antikrebsmittel in entartete Zellen einzuschleusen. Unter entartete Zellen werden onkogene Zellen, Tumorzellen als auch krebszellen verstanden, also Zellen, welche vollständig entartet sind oder sich auf dem Weg zur vollständigen Entartung befinden. Als entartete Zellen werden somit Zellen bezeichnet, welche eine unkontrollierte Proliferation besitzen. Dies bedeutet, dass die therapeutischen Substanzen insbesondere Antikrebsmittel bei gleichzeitiger Anwesenheit der hierin beschriebenen Nanopartikel deutlich besser von den entarteten Zellen aufgenommen werden als bei Abwesenheit der Nanopartikel. Durch die verbesserte Aufnahme der therapeutischen Substanzen in die entarteten Zellen erfolgt sowohl eine deutliche Steigerung der Aktivität dieser Substanzen insbesondere Antikrebsmittel als auch eine Reduktion der Nebenwirkungen dieser Substanzen.

Unter Steigerung der Aktivität wird verstanden, dass dieselbe Menge an therapeutisch wirksamer Substanz, insbesondere Antikrebsmittel eine gesteigerte Wirksamkeit bei Anwesenheit der Nanopartikel als bei deren Abwesenheit zeigt. Unter Reduktion der Nebenwirkungen von therapeutischen Substanzen, insbesondere Antikrebsmitteln soll verstanden werden, dass die Schädigung gesunder Zellen bei gleicher Wirksamkeit oder bei derselben Menge an Antikrebsmittel geringer ist bei gleichzeitiger Anwesenheit der Nanopartikel als bei deren Abwesenheit.

Somit ist Voraussetzung für die Wirksamkeitssteigerung, dass die therapeutische Substanz im Verlaufe der großvolumigen Einschleusung der Nanopartikel mit in die Zelle aufgenommen werden kann. Offensichtlich führt die Invagination der Zellmembran infolge der Partikelbindung zumindest zur teilweisen Aufhebung der transmembranären Passagekontrolle und damit zu einem vollkommen neuen Einschleusungskanal für therapeutisch wirksame Substanzen. Dabei ist es vorteilhaft, wenn eine lokale Konzentrationserhöhung von Nanopartikeln und therapeutischer Substanz insbesondere Antikrebsmittel im Interstitium erreicht werden kann. Dies kann z.B. durch interstitielle Gabe der Mischung erreicht werden oder durch geeignete Anreicherungsstrategien, wie der kontrollierten Freisetzung (controlled release), der Erkennung von Rezeptoren oder auch anderen Biomolekülen, die von Liganden an der Partikeloberfläche erkannt werden (Targeting). Ebenfalls ist es möglich, nach interstitieller Gabe der Nanopartikel, die therapeutische Substanz systemisch zu verabreichen. Die intrazelluläre Aufnahme der Nanopartikel vollzieht sich innerhalb von Stunden bis Tagen, so dass auch eine mehrfache Gabe der Substanz innerhalb der Endozytose-Phase möglich ist. Eine systemische Gabe der Substanz ist insbesondere dann notwendig, wenn eine Metabolisierung erfolgen muss.

Die Nanopartikel sollten eine positive Oberflächenladung aufweisen, da derartige Nanopartikel besonders gut in entartete Zellen, insbesondere Krebszellen aufgenommen werden.

Eine unter physiologischen Bedingungen positive Oberflächenladung auf den Nanopartikeln lässt sich erreichen, indem man die Nanopartikel mit einer positiv polarisierbaren und/oder positiv ionisierbaren Beschichtung versieht.

Eine solche positiv polarisierbare und/oder positiv ionisierbare Beschichtung kann erhalten werden, indem man die Nanopartikel mit positiv polarisierbaren und/oder positiv ionisierbaren Stoffen beschichtet. Solche Stoffe können beispielsweise Aminogruppen oder protonierbare Stickstoffatome enthalten, welche beim entsprechenden pH-Wert in protonierter Form vorliegen.

Unter positiver Oberflächenladung soll eine positiv geladene Oberfläche oder eine positiv aufladbare Oberfläche oder eine positiv polarisierbare Oberfläche eines jeden Nanopartikels verstanden werden, wobei die Nanopartikel unter physiologischen Bedingungen diese positiv polarisierte oder positiv geladene Oberfläche aufweisen sollten.

Die Nanopartikel besitzen in einer bevorzugten Ausführungsform eine Beschichtung aus polykondensierten Aminosilanen und eventuell eine weitere die positiven Ladungen kompensierende Carboxylatgruppen aufweisende äußere Beschichtung.

Da die positiv geladene oder positiv polarisierbare oder positiv aufladbare Beschichtung nur langsam biologisch abgebaut werden sollte, besteht sie vorzugsweise aus biologisch stabilen bzw. biologisch inerten bzw. biostabilen Substanzen, beispielsweise aus Polymeren.

Als biostabile Polymere können eingesetzt werden: Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseäcetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate. Chitosane und Copolymere und/oder Mischungen dieser Substanzen.

Die biologisch stabilen Polymere sollten eine genügende Anzahl positiver oder positiv polarisierbarer oder positiv aufladbarer Gruppen wie beispielsweise Aminogruppen oder Stickstoffatome aufweisen. Normalerweise verfügt die positiv geladene Beschichtung pro Nanopartikel im Durchschnitt über mindestens 50, bevorzugt mindestens 100 und insbesondere bevorzugt mindestens 500 kationische, positiv polarisierbare und/oder positiv aufladbare Gruppen wie beispielsweise Aminogruppen.

Bei bevorzugten Ausführungsformen besteht diese Beschichtung aus monomeren Aminosilanen wie z.B. 3-Aminopropyltriethoxysilan, 2-Aminoethyl-3-aminopropyltrimethoxysilan, Trimethoxysilylpropyldiethylentriamin oder N-(6-Aminohexyl)-3-aminopropyltrimethoxysilan, welche zur Erzielung der erforderlichen Stabilität nach bekannten Verfahren polykondensiert werden. Ein geeignetes Verfahren ist beispielsweise in DE 19614136 A oder DE 19515820 A beschrieben.

Die positiv geladene Schicht oder Beschichtung kann zur Kompensierung der Ladung als auch zur Steigerung der Zelldiskriminierung mit einer weiteren Beschichtung aus vorzugsweise biologisch abbaubaren Polymeren bzw. biodegradierbaren Stoffen überzogen werden.

Als bioabbaubare Polymere werden vorzugsweise verwendet: Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-ß-Maleinsäure Polycaprolactonbutylacrylate, Multiblockpolymere wie z.B. aus Oligocaprolactondiole und Oligodioxanondiole, Polyetherestermultiblockpolymere wie z.B. PEG und Poly(butylenterephtalat), Polypivotolactone, Polyglycolsäuretrimethylcarbonate Polycaprolacton-glycolide, Poly(g-ethylglutamat), Poly(DTH-iminocarbonat), Poly(DTE-co-DT-carbonat), Poly(bisphenol A-iminocarbonat), Polyorthoester, Polyglycolsäuretrimethylcarbonate, Polytrimethylcarbonate Polyiminocarbonate, Poly(N-vinyl)-pyrrolidon, Polyvinylalkohole, Polyesteramide, glycolierte Polyester, Polyphosphoester, Polyphosphazene, Poly[(p-carboxyphenoxy)propan] Polyhydroxypentansäure, Polyanhydride, Polyethylenoxidpropylenoxid, weiche Polyurethane, Polyurethane mit Aminosäureresten im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Alginate, Glycosaminoglycane, Saccharide, Polysaccharide, Proteoglykane, Glycoproteine, Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen.

Insbesondere bevorzugt sind Polymere oder Copolymere auf Basis von α-Hydroxycarbonsäuren wie beispielsweise Polymilchsäure, Polylactide, Polyglycolsäure, Polyglycolide sowie Copolymere davon. Zudem bevorzugt sind Polyole (z.B. Polyethylenglycol) und Polysäure wie z.B. Polyacrylsäuren als auch Kohlenhydrate und Zucker, insbesondere Dextrane. Die Nanopartikel können ferner noch mit einer dritten Beschichtung versehen bzw. überzogen. Die Beschichtungen können als Schutzhülle, Barriereschicht oder zur Zelldiskriminierung dienen.

Eine zellspezifische Beschichtung erhöht die Affinität der Nanopartikel zu bestimmten Zellen, beispielsweise zu bestimmten Bakterienzellen oder zu bestimmten Tumorzellen und dient somit der Zelldiskriminierung. Solche zellspezitischen Nanopartikel reichem sich bevorzugt in solchen Zellen an, zu denen sie aufgrund der Funktionalität auf ihrer Oberfläche eine erhöhte Affinität haben und sind somit tumorspezifisch. Mit dieser Technologie lassen sich beispielsweise tumorspezifische Nanopartikel für bestimmte Krebsarten entwickeln.

Zur weiteren Affinitätssteigerung bezüglich bestimmter Zellen können auf der Oberfläche der Nanopartikel bzw. auf der äußeren Schicht oder Hülle der Nanopartikel polyklonale, Antikörper, monoklonale Antikörper, humanifizierte Antikörper, humane Antikörper, chimere Antikörper, rekombinante Antikörper, bispezifische Antikörper, Antikörperfragmente, Aptamere, Fab-Fragmente, Fc-Fragmente, Peptide, Peptidomimetika, gap-Mere, Ribozymes, CpG-Oligomere, DNA-Zyme, Riboswitches und/oder Lipide gekoppelt und/oder angelagert und/oder eingelagert werden. Die Verbindungen sind derart ausgestaltet, dass sie bestimmte Zellen beispielsweise Tumorzellen erkennen und die Zelldiskriminierung der Nanopartikel weiter steigern.

Die Nanopartikel selbst bestehen vorzugsweise aus einem magnetischen Material, einem ferromagnetischen, antiferromagnetischen, ferrimagnetischen, antiferrimagnetischen oder superparamagnetischen Material, weiter bevorzugt aus Eisenoxiden, insbesondere superparamagnetischen Eisenoxiden oder aus reinem Eisen, welches mit einer Oxidschicht versehen ist. Derartige Nanopartikel können durch ein magnetisches Wechselfeld erwärmt werden. Eine Erwärmung des die Nanopartikel enthaltenden Gewebes auf über 50°C ist möglich. Derartig hohe Temperaturen können erreicht werden, da bis zu 1000 pg und mehr Eisen in Form der Nanopartikel pro Tumorzelle aufgenommen werden können.

Die Nanopartikel bestehen vorzugsweise aus Eisenoxiden und insbesondere aus Magnetit (Fe₃O₄), Maghemit (γ-Fe₂O₃) oder Mischungen dieser beiden Oxide und sind vorzugsweise superparamagnetisch. Allgemein können die bevorzugten Nanopartikel durch die Formel FeOx wiedergegeben werden, worin X eine Zahl von 1 bis 2 bedeutet. Es besteht aber auch die Möglichkeit, die Nanopartikel in ein nicht magnetisches Material wie beispielsweise Siliziumoxid (SiO₂) einzulagern (s.u.). Die Nanopartikel weisen vorzugsweise einen Durchmesser von weniger als 500 nm auf. Vorzugsweise besitzen die Nanopartikel einen durchschnittlichen Durchmesser von 15 nm oder liegen vorzugsweise in dem Größenbereich von 1 - 100 nm und insbesondere bevorzugt im Bereich von 10 - 20 nm.

Neben den magnetischen Materialien der Formel FeOₓ, worin X eine Zahl im Bereich von 1,0 bis 2,0 ist, sind erfindungsgemäß auch Materialien der allgemeinen Formel M(II)Fe₂O₄ mit M = Co, Ni, Mn, Zn, Cu, Cd, Ba oder andere Ferrite einsetzbar. Der Gehalt an von Eisenatomen verschiedenen Metallatomen beträgt vorzugsweise nicht mehr als 70 Metallatom-%, insbesondere nicht mehr als 35 Metallatom-%. Bevorzugt bestehen die Nanopartikel jedoch zu über 98 Gew.-% aus Eisenoxid, welches sowohl Fe(III) als auch Fe(II) in einem Verhältnis von vorzugsweise 1 zu 1 bis 1 zu 3 enthält. Ferner eignen sich auch Silica- oder Polymerpartikel, in die magnetische Materialien wie beispielsweise die hierin genannten magnetischen Materialien eingelagert und/oder angebunden sind.

Die verwendeten Nanopartikel-Kerne können auch aus nichtmagnetischen Materialien bestehen. In Frage kommen z.B. Nanopartikel aus Polymeren (z.B. PLGA, Polyacrylamid, Polybutylcyanoacrylat), Metallen sowie aus sämtlichen oxidischen Materialien (z.B. MgO, CaO, TiO2, ZrO₂, SiO₂, Al₂O₃). Geeignet ist jedes Material, dass sich mit den oben beschriebenen Methoden mit tumorspezifischen Hüllen beschichten lässt, da die Endozytosefähigkeit nicht vom Kern der Partikel, sondern von der Hülle abhängt.

Die Nanopartikel oder nanoskaligen Teilchen besitzen vorzugsweise einen durchschnittlichen Teilchendurchmesser von nicht mehr als 100 nm, bevorzugt nicht mehr als 50 nm und insbesondere bevorzugt nicht mehr als 30 nm. Der mittlere Teilchendurchmesser liegt vorzugsweise bei 1 - 40 nm, bevorzugt bei 3 - 30 nm und insbesondere bevorzugt bei 5 - 25 nm.

Derartige Nanopartikel eignen sich überraschenderweise sehr gut zur Einschleusung von therapeutischen Substanzen in bestimmte Zelltypen, wodurch die Wirksamkeit der therapeutischen Substanzen deutlich gesteigert wird. Bei diesen therapeutischen Substanzen handelt es sich vorzugsweise um Antikrebsmittel, Zytostatika, zytotoxische Wirkstoffe, antiproliverative Wirkstoffe, antiphlogistische Wirkstoffe, antimigrative Wirkstoffe, antiangiogene Wirkstoffe, antiinflammatorische Wirkstoffe, antibakterielle Wirkstoffe und/oder Mikrotubuli-Inhibitoren.

Als zytotoxische und/oder zytostatische Verbindungen, d.h. chemische Verbindungen mit zytotoxischen und/oder zytostatischen Eigenschaften können unter anderem Alkylierungsmittel, Antibiotika mit zytostatischen Eigenschaften, Antimetabolite, Mikrotubuli-Inhibitoren und Topoisomerase-Inhibitoren, Platin-enthaltende Verbindungen und andere Zytostatika wie beispielsweise Asparaginase, Tretinoin, Alkaloide, Podophyllotoxine, Taxane und Miltefosin^{®}, Hormone, Immunmodulatoren, monoklonale Antikörper, Signaltransduktoren (Signaltransduktionsmoleküle) und Zytokine eingesetzt werden.

Als Beispiele für Alkylierungsmittel können unter anderem Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin und Nimustin genannt werden.

Beispiele für Antibiotika mit zytostatischen Eigenschaften sind Daunorubicin als auch liposomales Daunorubicin, Doxorubicin (Adriamycin), Dactinomycin, Mitomycin C, Bleomycin, Epirubicin (4-Epi-Adriamycin), Idarubicin, Dactinomycin, Mitoxantron, Amsacrin und Actinomycin D.

Methotrexat, 5-Fluorouracil, 6-Thioguanin, 6-Mercaptopurin, Fludarabin, Cladribin, Pentostatin, Gemcitabin, Cytarabin, Azathioprin, Raltitrexed, Capecitabin, Cytosinarabinosid, Tioguanin und Mercaptopurin können als Beispiele für Antimetabolite (antimetabolische Wirkstoffe) angeführt werden.

Zu der Klasse der Alkaloide und Podophyllotoxine gehören unter anderem Vincristin, Vinblastin, Vindesin, Etoposid als auch Teniposid. Des weiteren können Platin-enthaltende Verbindungen eingesetzt werden. Als Platin-enthaltende Verbindungen seien beispielsweise Cisplatin, Carboplatin und Oxaliplatin genannt. Zu den Mikrotubuli-Inhibitoren zählen beispielsweise Alkaloide wie beispielsweise Vinca-Alkaloide (Vincristin, Vinblastin, Vindesin, Venorelbin) und Paclitaxel (Taxol^{®}) sowie Derivate des Paclitaxel. Als Topoisomerase-Inhibitoren können beispielsweise Etoposid, Teniposid, Camptothecin, Topotecan und Irinotecan genannt werden.

Paclitaxel und Docetaxel sind Beispiele für die Verbindungsklasse der Taxane und zu den anderen zytostatischen Wirkstoffen (anderen Zytostatika) zählen beispielsweise Hydroxycarbamide (Hydroxyurea), Imatinib, Miltefosin^{®}, Amsacrin, Topotecan (Topoisomerase-I-Inhibitor), Pentostatin, Bexaroten, Tretinoin und Asparaginase. Vertreter der Verbindungsklasse der monoklonalen Antikörper sind unter anderem Trastuzumab (auch bekannt als Herceptin^{®}), Alemtuzumab (auch bekannt als MabCampath^{®}) und Rituximab (auch bekannt als MabThera^{®}). Auch können Hormone wie beispielsweise Glucocorticoide (Prednison), Oestrogene (Fosfestrol, Estramustin), LHRH (Buserelin, Goserelin, Leuprorelin, Triptorelin), Flutamid, Cyproteronacetat, Tamoxifen, Toremifen, Aminoglutethimid, Formestan, Exemestan, Letrozol und Anastrozol eingesetzt werden. Zu den Klassen der Immunmodulatoren, Zytokine, Antikörper und Signaltransduktoren zählen Interleukin-2, Interferon-α, Erythropoietin, G-CSF, Trastuzumab (Herceptin^{®}), Rituximab (MabThera^{®}), Efitinib (Iressa^{®}), Ibritumomab (Zevalin^{®}), Levamisol sowie Retinoide.

Als weitere mögliche therapeutische Substanzen sind zu nennen: Actinomycin D, Aminoglutethimid, Anthracycline, Aromatasehemmer, Antiöstrogene, Buselerin, Folsäureantagonisten, Goselerin, Hormonantagonisten, Hycamtin, Hydroxyharnstoff, Mitosehemmstoffe, Tamoxifen, Testolacton, Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, 4-Hydroxyoxycyclophosphamid, Tropfosfamid, Bendamustin, Thymosin α-1, Aclarubicin, Fludarabin-5'-dihydrogenphosphat, Antagonisten von Purin- und Pyrimidin-Basen, Hydroxycarbamid, Aldesleukin, Pegasparase, Letrozol, Aminoglutethemid, Adriamycin, Azithromycin, Spiramycin, Cepharantin, Epothilone A und B, Mitoxanthrone, Azathioprin, Mycophenolatmofetil, c-myc-Antisense, b-myc-Antisense, Betulinsäure, Camptothecin, Camptothecin-Derivate, Melanocyte-stimulating hormon (α-MSH), aktiviertes Protein C, IL1-β-Inhibitor, Fumarsäure und deren Ester, Dermicidin, Calcipotriol, Tacalcitol, Lapachol, β-Lapachon, Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Chephalomannin, Trastuzumab, Exemestan, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren, Pentaerythrityltetranitrat, Syndnoeimine, S-Nitrosoderivate, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazol, Bacitracin, Vitronectin-Rezeptor Antagonisten, Azelastin, freie Nukleinsäuren, Nukleinsäuren in Virenüberträger inkorporiert, DNA- und RNA-Fragmente, Plasminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline, Dicloxacillin, Oxacillin, Sulfonamide, Metronidazol, Antithrombotika, Argatroban, Aspirin, Abciximab, synthetisches Antithrombin, Bivalirudin, Coumadin, Enoxoparin, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon α, ß und γ, Histaminantagonisten, Serotoninblocker, Apoptoseinhibitoren, Apoptoseregulatoren, NF-kB oder Bcl-xL-Antisense-Oligonukleotide, Halofuginon, Nifedipin, Tocopherol Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron., natürliche und synthetisch hergestellte Steroide wie Bryophyllin A, Inotodiol, Maquirosid A, Ghalakinosid, Mansonin, Streblosid, Hydrocortison, Betamethason, Dexamethason, Fenoporfen, Ibuprofen, Indomethacin, Naproxen, Phenylbutazon, Acyclovir, Ganciclovir, Zidovudin, Antimykotika, Clotrimazol, Flucytosin, Griseofulvin, Ketoconazol, Miconazol, Nystatin, Terbinafin, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrosti-stachin, Ovatodiolide, 4,7-Oxycycloanisomelsäure, Baccharinoide B1, B2, B3 und B7, Tubeimosid, Bruceanole A,B und C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B. Erfindungswesentlich ist somit, dass die mindestens eine therapeutisch wirksame Substanz mit zellgängigen Nanopartikeln, die von Tumorzellen in hohem Maße durch Endozytose aufgenommen werden, verabreicht wird. Nanopartikel, wie sie beispielsweise auch in der DE 197 26 282 A beschrieben sind, werden von Tumorzellen im Vergleich zu Normalzellen vermehrt aufgenommen. Wie durch in-vitro Untersuchungen mit Eisenoxid-Nanpartikeln gezeigt werden konnte, werden in bestimmten Tumorzelllinien mehr als 1000 pg/Zelle Eisen in Form von Nanopartikeln aufgenommen. Dabei werden die Nanopartikel großvolumig in die Zellen eingeschleust, wie durch Elektronenmikroskopieuntersuchungen gezeigt werden konnte. Überraschend wurde nun durch in-vitro-Untersuchungen festgestellt, dass die Verabreichung von pharmazeutischen Zusammensetzungen aus diesen Nanopartikeln und mindestens einer therapeutisch wirksamen Substanz, insbesondere eines Zytostatikums oder Antikrebsmittels zu einer Wirksamkeitssteigerung der verabreichten Substanz führt. Dieser Effekt wurde auch beobachtet, wenn keine Bindung (kovalent, ionisch oder adsorptiv) zwischen den Nanopartikeln und dem Zytostatikum vorliegt. Eine Bindung zwischen Zytostatikum und Nanopartikel beeinflusst diese Ergebnisse nur dann, wenn das Zytostatikum durch die Bindungsreaktion seine Wirksamkeit verliert, oder wenn die Bindung so fest ist, dass nach intrazellulärer Aufnahme keine Freisetzung des Zytostatikums erfolgen kann.

Somit eignen sich die pharmazeutischen Zusammensetzungen aus den besagten Nanopartikeln und mindestens einer therapeutisch wirksamen Substanz hervorragend zur Prophylaxe und Behandlung von Krebserkrankungen, Geschwüren, Tumoren, Karzinomen sowie in der Proliferation gestörten Zellen.

Als Beispiele für Krebs- und Tumorarten, bei denen die Zusammensetzungen aus Nanopartikel und therapeutischer Substanz eingesetzt werden können, sind zu nennen: Adenokarzinome, Aderhautmelanom, Akute Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dunndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hals-, Nasen- und Ohrentumoren, Hämatologische Neoplasien, Haarzell-Leukämie, Harnröhrenkrebs, Hautkrebs, Hirntumoren (Gliome), Hirnmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals- Nasen- und Ohrenbereichs), Kolonkarzinom, Kraniopharyngeome, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakarzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome. u. osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs.

Bevorzugt sind insbesondere solide Tumoren. Ferner sind bevorzugt Prostatakarzinome, Gehirntumore, Sarkome, Zervix-Karzinome, Ovarialkarzinome, Mammakarzinome, Bronchialkarzinome, Melanome, Kopf-Hals Tumore, Ösophaguskarzinome, Rektumkarzinome, Pankreas-, Blasen- und Nierenkarzinome, Metastasen der Leber, des Gehirns und in Lymphknoten.

Insbesondere bevorzugt ist ferner die Anwendung und der Einsatz der erfindungsgemäßen pharmazeutischen Zusammensetzungen zusammen mit der konventionellen Hyperthermie, Magnetflüssigkeitshyperthermie bzw. Thermotherapie mit magnetischen Flüssigkeiten, Strahlentherapie und/oder zusammen mit der herkömmlichen Chemotherapie. Die erfindungsgemäßen Zusammensetzungen unterstützen somit vorzugsweise auch die konventionellen Krebsbehandlungsmethoden.

Demgemäß ist die vorliegende Erfindung auch auf Kombinationen aus einer erfindungsgemäßen pharmazeutischen Zusammensetzung und Hyperthermie, Thermotherapie, Strahlentherapie und/oder Chemotherapie gerichtet.

Als Beispiel für eine derartige Kombination sei die Verwendung einer erfindungsgemäßen pharmazeutischen Zusammensetzung zusammen mit der Magnetflüssigkeitshyperthermie bzw. Thermotherapie mit magnetischen Flüssigkeiten genannt. Dabei wirkt ein wechselndes Magnetfeld als externe Anregung, die im Falle von superparamagnetischen Nanopartikeln verschiedene Relaxationsprozesse der Nanopartikel in Gang setzt. Diese Prozesse führen u.A. zu einer Erwärmung der Nanopartikel und ihrer Umgebung. Diese durch das magnetische Wechselfeld in Gang gesetzten Prozesse werden erfindungsgemäß dazu benutzt, die entarteten Zellen zu erwärmen, wodurch die therapeutisch wirksame Substanz noch schneller zu einer Abtötung dieser Zelle führen kann.

Somit werden die pharmazeutischen Zusammensetzungen für die Behandlung als auch zur Prophylaxe von Krankheiten eingesetzt, welche sich durch entartete Zellspezies oder körperfremde Zellen auszeichnen und bei denen die Eigenschaften der erfindungsgemäßen Nanopartikel ausgenutzt werden können, zum einen zwischen fremden Zellen bzw. entarteten Zellen und gesunden körpereigenen Zellen zu diskriminieren und zum anderen therapeutisch wirksame Substanzen in diese Zellen einzuschleusen. Als entartete Zellen gelten insbesondere Krebszellen bzw. in ihrer Proliferation gestörte Zellen als auch stenotisches oder restenotisches Gewebe. Als körperfremde Zellen können insbesondere Bakterien genannt werden.

Durch die Eigenschaft der Nanopartikel, Wirkstoffe in entartete Zellen einschleusen zu können, wird die Wirksamkeit dieser Wirkstoffe gesteigerte. Kombiniert man zudem die Applikation der pharmazeutischen Zusammensetzung umfassend Nanopartikel und therapeutisch wirksame Substanz mit der Strahlentherapie oder Chemotherapie oder der Hyperthermie oder Hyperthermie und Chemotherapie oder Hyperthermie und Strahlentherapie, dann kann die Effektivität der Behandlung ein weiteres Mal gesteigert werden.

Erfindungsgemäß wird somit die Effektivität der Wirkstoffe infolge einer erhöhten lokalen, lokoregionalen oder intrazellulären Wirkstoffkonzentration gesteigert und die systemische Toxizität sowie Nebenwirkungen der therapeutisch wirksamen Substanz gemindert.

### Figurenbeschreibung

- Figur 1: zeigt RUSIRS1 Zellen 3h nach Zugabe von Mitomycin in 0,9% NaCl
- Figur 2: zeigt RUSIRS1 Zellen 3h nach Zugabe von Mitomycin in 0,9% NaCl + Nanopartikel
- Figur 3: zeigt RUSIRS1 Zellen 24h nach Zugabe von Mitomycin in 0,9% NaCl
- Figur 4: zeigt RUSIRS1 Zellen 24h nach Zugabe von Mitomycin in 0,9% NaCl + Nanopartikel
- Figur 5: zeigt RUSIRS1 Zellen 48h nach Zugabe von Mitomycin in 0,9% NaCl
- Figur 6: zeigt RUSIRS1 Zellen 48h nach Zugabe von Mitomycin in 0,9% NaCl + Nanopartikel
- Figur 7: zeigt RUSIRS1 Zellen 48h (Kontrolle)
- Figur 8: zeigt BT20 Zellen nach 72 Std. als Kontrolle mit Cefamandol jedoch ohne Nanopartikel
- Figur 9: zeigt BT20 Zellen nach 72 Std. Inkubation mit Nanopartikeln und Cefamandol
- Figur 10: zeigt BT20 Zellen nach 72 Std. Inkubation mit Nanopartikeln und Cefamandol
- Figur 11: zeigt WiDr Zellen nach 72 Std. als Kontrolle mit Cefamandol jedoch ohne Nanopartikel
- Figur 12: zeigt WiDr Zellen nach 72 Std. Inkubation mit Nanopartikeln und Cefamandol

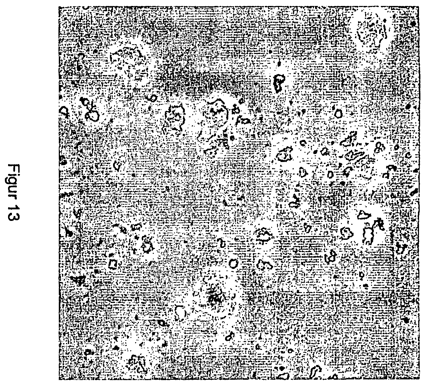

- Figur 13: zeigt WiDr Zellen nach 72 Std. Inkubation mit Nanopartikeln und Cefamandol

### Beispiele

### Beispiel 1:

### Wirksamkeitssteigerung des Zytostatikums Mitomycin (in-vitro)

Die Steigerung der Wirksamkeit von Mitomycin zur Bekämpfung von Tumorzellen ließ sich durch in-vitro Untersuchungen belegen. Die in-vitro Untersuchungen wurden mit der Glioblastom-Human-Zelllinie RUSIRS1 (Himtumor) durchgeführt. Die Glioblastömzellen wurden wie in DE 199 12 798 C1 beschrieben aus dem Tumorgewebe eines Patienten entnommen und kultiviert. Zur Testung der Wirksamkeit des Mitomycin/Nanopartikel-Gemisches wurden jeweils 2x10⁶ RUSIRS1 Zellen in einer 75 qcm Zellkulturflasche mit 25 ml Zellkulturmedium (D-MEM + 20% FBS + 1,2 ml Pyruvat) angesetzt. Zu dieser Zellsuspension wurden 136 µl Magnetflüssigkeit MFL AS M01 (Eisenoxid-Nanopartikel mit einer Beschichtung aus polykondensiertem N-(2-Aminoethyl)-3-(trimethoxysilyl)propylamin, Hersteller: MagForce Nanotechnologies GmbH, Berlin) (c_{Fe} = 2 mol/l) und 390 µl Mitomycin-Lsg. (1 mg/ml in 0.9 % NaCl) gegeben. Die Proben wurden vor der Zugabe zu den Zellen für 15 Minuten auf 37°C erwärmt und 10 Minuten bei RT stehen gelassen. Eine Vergleichsprobe mit Mitomycin, aber ohne Nanopartikel wurde auf die gleiche Weise angesetzt.

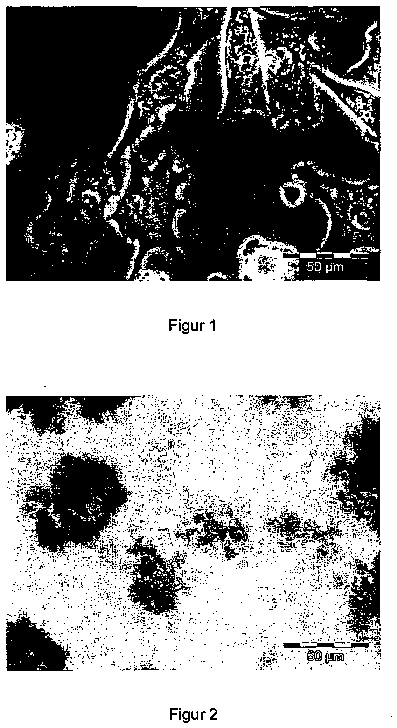

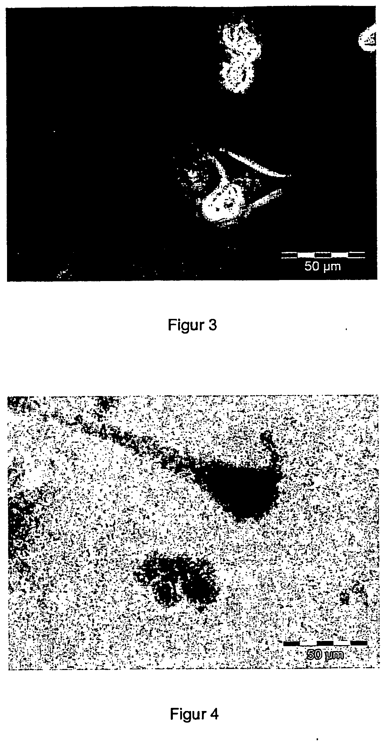

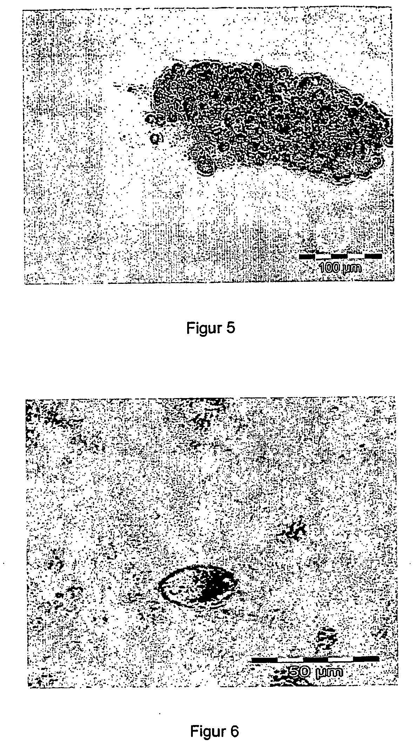

Der Einfluss der Nanopartikel auf die Wirksamkeit des Mitomycins lässt sich anhand der Abbildungen 1-6 erläutern. Zellen, die nur mit einer Mitomycin-Lsg. versetzt worden sind, zeigen erst nach 48 Stunden Inkubationszeit eine deutliche Schädigung. Im Gegensatz dazu zeigen Zellen, die mit dem Zytostatikum und den Partikeln inkubiert wurden, eine deutliche Schädigung schon nach 3 Stunden. Die Aufnahme der Eisenoxid-Nanopartikel in die Zellen lässt sich anhand der Braunfärbung der Zelle belegen. Vergleichsversuche haben gezeigt, dass die Nanopartikel alleine (ohne Mitomycin) ebenfalls aufgenommen werden, aber nicht zu einer derart hohen Zellschädigung führen. Eine schnelle Zellschädigung (nach 3h) tritt also nur auf, wenn Partikel und Mitomycin gleichzeitig vorhanden sind. Durch die Endozytose der Partikel wurde also auch Mitomycin eingeschleust, was zu der signifikanten Zellschädigung führte.

### Beispiel 2:

### Wirksamkeitssteigerung des Antibiotikums Cefamandol (in-vitro)

Cefamandol (CAS Nr. 30034-03-8) wird zur Bekämpfung von bakteriellen Infektionen eingesetzt. Eine Wirksamkeit gegen Krebszellen wurde im Jahr 2000 überraschenderweise an Biopsie-Material von Lebermetastasen gefunden (MagForce Nanotechnologies). Das Potential dieser Substanz zur Bekämpfung von Krebszellen ist allerdings als eher gering einzustufen. Unsere Untersuchungen haben ergeben, dass eine Schädigung von Tumorzellen (in-vitro) in der Regel erst ab einer Konzentration von 0,5 mg/ml (Konz. im Zellkulturmedium) einsetzt. Die Wirksamkeit von Cefamandol zur Bekämpfung von Tumorzellen lässt sich jedoch bei gleichzeitiger Anwendung von Nanopartikeln drastisch erhöhen.

Die in-vitro Untersuchungen wurden mit den Zelllinien BT20 (Mamma-Karzinom) und WiDr (Kolon-Karzinom) durchgeführt. Die Tumorzellen wurden wie in DE 199 12 798 C1 beschrieben aus dem Tumorgewebe eines Patienten entnommen und kultiviert. Zur Überprüfung der Wirksamkeit des Cefamandol/Nanopartikel-Gemisches wurden jeweils 2x10⁶ Zellen in einer 75 qcm Zellkulturflasche mit 25 ml Zellkulturmedium (RPMI + 10% FBS + 1,2 ml Pyruvat für WiDr-Zellen, bzw. BME + 10% FBS + Pyruvat + 5 ml non. ess. AS + 5 ml Glutamin für BT20-Zellen) angesetzt. Zu dieser Zellsuspension wurden 136 µl Magnetflüssigkeit MFL AS M01 (Eisenoxid-Nanopartikel mit einer Beschichtung aus polykondensiertem N-(2-Aminoethyl)-3-(trimethoxysilyl)propylamin, (Hersteller: MagForce Nanotechnologies AG, Berlin) (c_{Fe} = 2 mol/l) und 390 µl Cefamandol-Lsg. (Stammlösung 1 mg/ml in 0.9 % NaCl) gegeben. Die Konzentration von Cefamandol im Zellkulturmedium (25 ml) betrug somit 0,016 mg/ml, also deutlich unterhalb der Wirksamkeitsschwelle des reinen Cefamandols.

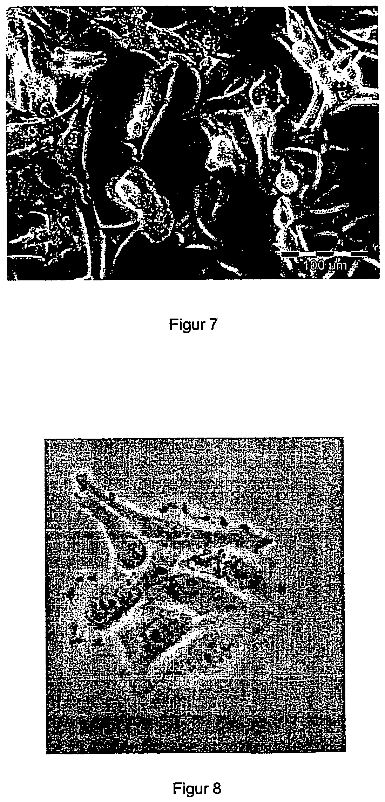

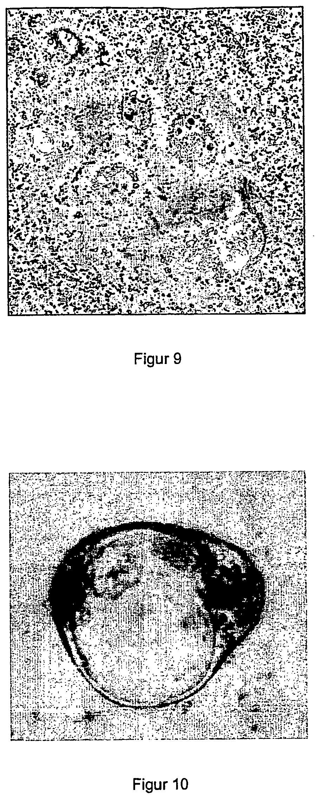

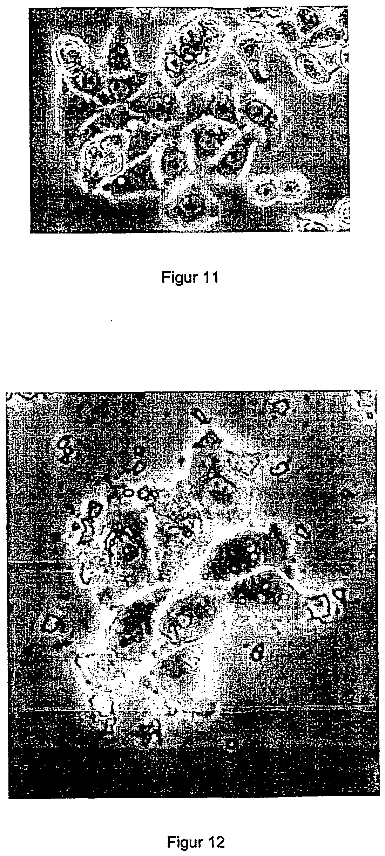

Nach 72 h Inkubation war eine deutliche Zellschädigung festzustellen, wie die Abbildungen 7-12 belegen. Im Fall von BT20 waren nach 72 Std. 30,5 % der Zellen tot, im Fall von WiDr 24%. Weder durch Cefamandol in der gewählten Konzentration, noch durch die Nanopartikel alleine kommt es zum Absterben von Zellen (0% tote Zellen). Erst durch die Kombination von Cefamandol und Nanopartikeln ergibt sich durch die Einschleusung des Cefamandols in die Zellen diese signifikante Schädigung der Tumorzellen.

### Beispiel 3:

### Herstellung einer pharmazeutischen Zusammensetzung aus Nanopartikeln, pharmakologischem Wirkstoff und Lösungsmittel

Zu einem ml einer wässrigen Dispersion superparamagnetischer Eisenoxid-Nanopartikel (Eisenkonzentration 2 mol/l) werden ca. 1 mg Zytostatikum (oder 1 bis 10 mmol, vorzugsweise 2 bis 6 mmol Zytostatikum) gegeben.

Sollte das Zytostatikum nicht ausreichend wasserlöslich sein, so können Cosolventien in einer Menge bis zu 20Vol.% der Lösung eingesetzt werden. Als Cosolventien können DMSO, DMS, Ethanol, Essigsäureethylester oder andere physiologisch verträgliche Lösungsmittel eingesetzt werden.

### Beispiel 4:

Zu einem ml der Magnetflüssigkeit MFL AS M01 (Eisenoxid-Nanopartikel mit einer Beschichtung aus polykondensiertem N-(2-Aminoethyl)-3-(trimethoxysilyl)propylamin, (Hersteller: MagForce Nanotechnologies AG, Berlin) (c_{Fe} = 2 mol/l) werden 1 mg Carmustin oder 1 mg Cisplatin oder 1 mg Epirubicin oder 1,5 mg Gemcitabin oder 1 mg Imatinib oder 0,8 mg Paclitaxel oder 1,2 mg Vinblastin oder 1 mg Vincristin oder 1,5 mg Adriamycin oder 1 mg Oxacillin oder 1 mg Tetracyclin oder 1 mg Temozolomid gegeben und durchmischt.

### Beispiel 5:

### Wirksamkeitssteigerung des Zytostatikums Mitoxantron (in-vitro)

Primäre Prostata-Karzinom-Zellen wurden wie in DE 199 12 798 C1 beschrieben kultiviert. Zur Überprüfung der Wirksamkeit eines Mitoxantron/Nanopartikel-Gemisches wurden jeweils 2x10⁶ Zellen in einer 75 qcm Zellkulturflasche mit 25 ml Zellkulturmedium angesetzt. Zu dieser Zellsuspension wurden 136 µl Magnetflüssigkeit MFL AS M01 (Eisenoxid-Nanopartikel mit einer Beschichtung aus polykondensiertem N-(2-Aminoethyl)-3-(trimethoxysilyl)propylamin, (Hersteller: MagForce Nanotechnologies AG, Berlin) (c_{Fe} = 2 mol/l) und 390 µl Mitoxantron-Lsg. (Stammlösung 1 mg/ml in 0.9 % NaCl) gegeben. Nach 72 h Inkubation war eine deutliche Zellschädigung festzustellen. Ein ähnlicher Effekt lässt sich auch mit den Zytostatika Epirubicin und Docetaxel (gelöst in polyoxyethyliertem Sorbitol; Polysorbat 80) belegen.

### Beispiel 6:

### Wirksamkeitssteigerung des Zytostatikums 5-Fluorouracil (in-vitro)

Primäre Rektum-Karzinom-Zellen wurden wie in DE 199 12 798 C1 beschrieben kultiviert. Zur Überprüfung der Wirksamkeit eines 5-Fluorouracil/Nanopartikel-Gemisches wurden jeweils 2x10⁶ Zellen in einer 75 qcm Zellkulturflasche mit 25 ml Zellkulturmedium angesetzt. Zu dieser Zellsuspension wurden 136 µl Magnetflüssigkeit MFL AS M01 (Eisenoxid-Nanopartikel mit einer Beschichtung aus polykondensiertem N-(2-Aminoethyl)-3-(trimethoxysilyl)propylamin, (Hersteller: MagForce Nanotechnologies AG, Berlin) (c_{Fe} = 2 mol/l) und 390 µl 5-Fluorouracil_Lsg. (Stammlösung 1 mg/ml in 0.9 % NaCl) gegeben. Nach 72 h Inkubation war eine deutliche Zellschädigung festzustellen. Ein ähnlicher Effekt lässt sich auch mit den Zytostatika Irinotecan, und Oxaliplatin belegen.

### Beispiel 7:

### Wirksamkeitssteigerung des Zytostatikums Carboplatin (in-vitro)

Primäre Bronchialkarzinom-Zellen (nicht kleinzelliges Bronchialkarzinom; NSCLC) wurden wie in DE 199 12 798 C1 beschrieben kultiviert. Zur Überprüfung der Wirksamkeit eines Caboplatin/Nanopartikel-Gemisches wurden jeweils 2x10⁶ Zellen in einer 75 qcm Zellkulturflasche mit 25 ml Zellkulturmedium angesetzt. Zu dieser Zellsuspension wurden 136 µl Magnetflüssigkeit MFL AS M01 (Eisenoxid-Nanopartikel mit einer Beschichtung aus polykondensiertem N-(2-Aminoethyl)-3-(trimethoxysilyl)propylamin, (Hersteller: MagForce Nanotechnologies AG, Berlin) (c_{Fe} = 2 mol/l) und 390 µl Carboplatin-Lsg. (Stammlösung 1 mg/ml in 0.9 % NaCl) gegeben. Nach 72 h Inkubation war eine deutliche Zellschädigung festzustellen. Ein ähnlicher Effekt lässt sich auch mit den Zytostatika Gemcytabin und Docetaxel (gelöst in polyoxyethyliertem Sorbitol; Polysorbat 80) belegen.

### Beispiele 8 - 196:

Entsprechend der Versuchsdurchführung gemäß Beispiel 2 wurden die 7 Zelllinien a) Glioblastom-Human-Zelllinie RUSIRS1, b) Mamma-Karzinom Zelllinien BT20, c) Kolon-Karzinom Zellline WiDr, d) Bronchial-karzinom-Zellen NSCLC, e) Rektum-Karzinom-Zellen und f) Prostata-karzinom Zellline DU145 mit den in folgender Tabelle 1 genannten Wirkstoffen in-vitro getestet.

In allen Fällen zeigte sich eine Aktivitätssteigerung des Zytostytikums. Die aktivitätssteigernde Wirkung wird hinter dem jeweiligen Zytostatikum in Klammern angegeben, wobei (+) eine Steigerung von ca. 5% bis 80% und (++) eine Aktivitätssteigerung von 80% bis 500% bedeutet.

**Taqbelle 1: Aktivitätssteigerung bei Zytostatika**

| Glioblasto m-Human-Zelllinie RUSIRS1 | Mamma-Karzinom Zelllinien BT20 | Kolon-Karzinom Zellline WiDr | Bronchialkarzinom-Zellen NSCLC | Rektum-Karzinom-Zellen | Prostata-karzinom Zellline DU 145 | Neurogliom Zellline H4 |
|---|---|---|---|---|---|---|
| Letrozol (+) | Letrozol (+) | Letrozol (+) | Letrozol (+) | Letrozol (+) | Letrozol (+) | Letrozol (+) |
| Tamoxifen (+) | Tamoxifen (+) | Tamoxifen (++) | Tamoxifen (+) | | Tamoxifen (+) | Tamoxifen (+) |
| Somatostatin (+) | | Somatostatin (+) | Somatostatin (+) | Somatostatin (+) | Somatostatin (++) | Somatostatin (+) |
| Tacrolimus (+) | Tacrolimus (+) | | Tacrolimus (+) | Tacrolimus (+) | Tacrolimus (+) | Tacrolimus (++) |
| Ascomycin (++) | Ascomycin (+) | Ascomycin (+) | Ascomycin (+) | Ascomycin (+) | Ascomycin (+) | |
| Cerivastatin (+) | Cerivastatin (+) | Cerivastatin (+) | Cerivastatin (+) | Cerivastatin (++) | Cerivastatin (+) | Cerivastatin (+) |
| Simvastatin (+) | Simvastatin (+) | Simvastatin (+) | Simvastatin (++) | Simvastatin (+) | Simvastatin (+) | Simvastatin (+) |
| Bendamustin (+) | Bendamustin (+) | | Bendamustin (+) | | Bendamustin (+) | |
| Tobramycin (+) | Tobramycin (+) | Tobramycin (+) | Tobramycin (+) | Tobramycin (+) | Tobramycin (+) | Tobramycin (+) |
| Ganciclovir (++) | Ganciclovir (+) | Ganciclovir (+) | Ganciclovir (+) | Ganciclovir (+) | Ganciclovir (+) | Ganciclovir (+) |
| Acyclovir (+) | Acyclovir (+) | Acyclovir (+) | Acyclovir (+) | Acyclovir (++) | Acyclovir (+) | Acyclovir (+) |
| Ibuprofen (+) | Ibuprofen (+) | Ibuprofen (++) | Ibuprofen (+) | Ibuprofen (+) | Ibuprofen (+) | Ibuprofen (+) |
| Paclitaxel (+) | Paclitaxel (++) | Paclitaxel (+) | Paclitaxel (+) | Paclitaxel (+) | Paclitaxel (+) | Paclitaxel (+) |
| Diclofenac (+) | | Diclofenac (+) | Diclofenac (+) | Diclofenac (++) | Diclofenac (+) | Diclofenac (+) |
| Azelastin (+) | Azelastin (+) | | | Azelastin (+) | Azelastin (+) | Azelastin (+) |
| Oxacillin (+) | Oxacillin (+) | Oxacillin (++) | Oxacillin (+) | Oxacillin (+) | Oxacillin (++) | Oxacillin (+) |
| Nifedipin (+) | Nifedipin (+) | Nifedipin (+) | Nifedipin (+) | | Nifedipin (+) | Nifedipin (+) |
| Leflunomid (+) | Leflunomid (+) | Leflunomid (+) | Leflunomid (+) | Leflunomid (+) | Leflunomid (+) | Leflunomid (+) |
| Tetracyclin (+) | Tetracyclin (+) | Tetracyclin (+) | Tetracyclin (+) | Tetracyclin (++) | Tetracyclin (++) | Tetracyclin (+) |
| Rapamycin (+) | Rapamycin (++) | Rapamycin (+) | Rapamycin (+) | Rapamycin (++) | Rapamycin (+) | Rapamycin (+) |
| Imatinib (+) | Imatinib (+) | Imatinib (++) | Imatinib (+) | Imatinib (+) | Imatinib (+) | Imatinib (+) |
| Vincristin (+) | Vincristin (++) | Vincristin (+) | Vincristin (++) | Vincristin (++) | Vincristin (+) | Vincristin (+) |
| Gemcitabin (+) | Gemcitabin (+) | Gemcitabin (+) | Gemcitabin (++) | Gemcitabin (+) | Gemcitabin (+) | Gemcitabin (+) |
| Cladribin (+) | Cladribin (+) | Cladribin (++) | Cladribin (++) | | Cladribin (+) | Cladribin (+) |
| Idarubicin (++) | Idarubicin (+) | Idarubicin (+) | Idarubicin (++) | Idarubicin (+) | Idarubicin (+) | Idarubicin (+) |
| Busulfan (+) | | Busulfan (+) | Busulfan (++) | | Busulfan (+) | Busulfan (+) |
| Chlorambucil (+) | Chlorambucil (+) | Colorambucil (++) | Chlorambucil (+) | Chlorambucil (+) | Chlorambucil (+) | Chlorambucil (++) |
| Carmustin (+) | Carmustin (+) | Carmustin (+) | Carmustin (++) | Carmustin (+) | Carmustin (+) | Carmustin (+) |
| Cisplatin (+) | Cisplatin (+) | Cisplatin (+) | Cisplatin (++) | Cisplatin (+) | Cisplatin (+) | Cisplatin (++) |

## Patentansprüche

1. Nanopartikel zur Verwendung in einem Verfahren zur Prophylaxe und/oder Behandlung von Krebserkrankungen, Geschwüren, Tumoren, Karzinomen oder in der Proliferation gestörten Zellen, wobei die Nanopartikel eine positive Oberflächenladung aufweisen, wobei die Nanopartikel derart zusammen mit einem Antikrebsmittel verabreicht werden, dass die Nanopartikel und das Antikrebsmittel gleichzeitig an der entarteten Zelle anwesend sind, und wobei die Nanopartikel und das Antikrebsmittel in getrennten Lösungen verabreicht werden.

2. Nanopartikel zur Verwendung nach Anspruch 1, **dadurch gekennzeichnet, dass** die Nanopartikel magnetische Nanopartikel sind, bevorzugt aus einem magnetischen Material, einem ferromagnetischen, antiferromagnetischen, ferrimagnetischen, antiferrimagnetischen oder superparamagnetischen Material.

3. Nanopartikel zur Verwendung nach einem der Ansprüche 1 bis 2, wobei die Nanopartikel eine Beschichtung aus positiv polarisierbaren und/oder positiv ionisierbaren Stoffen aufweisen.

4. Nanopartikel zur Verwendung nach Anspruch 3, wobei die positiv polarisierbaren und/oder positiv ionisierbaren Stoffe Aminogruppen und/oder Stickstoffatome aufweisen.

5. Nanopartikel zur Verwendung nach Anspruch 3 oder 4, wobei die Beschichtung aus biologisch stabilen bzw. biologisch inerten bzw. biostabilen Substanzen besteht, bevorzugt aus Polymeren, insbesondere aus polykondensierten Aminosilanen, insbesondere aus 3-Aminopropyltriethoxysilan, 2-Aminoethyl-3-aminopropyltrimethoxysilan, Trimethoxysilylpropyldiethylentriamin oder N-(6-Aminohexyl)-3-aminopropyltrimethoxysilan.

6. Nanopartikel zur Verwendung nach einem der vorherigen Ansprüche, wobei die Nanopartikel aus Eisenoxid, Magnetit, Maghemit oder M(II)Fe₂O₄ bestehen, worin M für Zn, Cu, Co, Ni, Cd, Ba oder Mn steht.

7. Nanopartikel zur Verwendung nach einem der vorherigen Ansprüche, wobei die Nanopartikel einen durchschnittlichen Durchmesser von 15 nm aufweisen oder im Größenbereich von 1 - 100 nm, bevorzugt im Bereich von 10 - 20 nm liegen.

8. Nanopartikel zur Verwendung nach einem der vorherigen Ansprüche, wobei die Nanopartikel im Rahmen einer Hyperthermie, Magnetflüssigkeitshyperthermie, Thermotherapie mit magnetischen Flüssigkeiten, Strahlentherapie und/oder Chemotherapie eingesetzt werden.

9. Nanopartikel zur Verwendung nach einem der vorherigen Ansprüche, wobei es sich bei dem Antikrebsmittel um ein Zytostatikum, zytotoxischen Wirkstoff, antiproliferativen Wirkstoff, antiphlogistischen Wirkstoff, antimigrativen Wirkstoff, antiangiogenen Wirkstoff, antiinflammatorischen Wirkstoff, antibakteriellen Wirkstoff oder einen Mikrotubuli-Inhibitor handelt.

10. Nanopartikel zur Verwendung nach einem der vorherigen Ansprüche, wobei das Antikrebsmittel ausgewählt wird aus der Gruppe umfassend: Actinomycin D, Aminoglutethimid, Amsacrin, Anastrozol, Antagonisten von Purin- und Pyrimidin-Basen, Anthracycline, Aromatasehemmer, Asparaginase, Antiöstrogene, Bexaroten, Bleomycin, Buselerin, Busulfan, Camptothecin-Derivate, Capecitabin, Carboplatin, Carmustin, Chlorambucil, Cisplatin, Cladribin, Cyclophosphamid, Cytarabin, Cytosinarabinosid, alkylierende Cytostatika, Dacarbazin, Dactinomycin, Daunorubicin, Docetaxel, Doxorubicin (Adriamycin), Doxorubicin lipo, Epirubicin, Estramustin, Etoposid, Exemestan, Fludarabin, Fluorouracil, Folsäureantagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Melphalan, Mercaptopurin, Methotrexat, Miltefosin, Mitomycine, Mitosehemmstoffe, Mitoxantron, Nimustine, Oxaliplatin, Paclitaxel, Pentostatin, Procarbazin, Tamoxifen, Temozolomid, Teniposid, Testolacton, Thiotepa, Thioguanin, Topoisomerase-Inhibitoren, Topotecan, Treosulfan, Tretinoin, Triptorelin, Trofosfamide, Vinblastin, Vincristin, Vindesin, Vinorelbin, zytostatisch wirksame Antibiotika, Actinomycin D, Aminoglutethimid, Anthracycline, Aromatasehemmer, Antiöstrogene, Buselerin, Folsäureantagonisten, Goselerin, Hormonantagonisten, Hycamtin, Hydroxyharnstoff, Letrozol, Mitosehemmstoffe, Tamoxifen, Testolacton, Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, 4-Hydroxyoxycyclophosphamid, Tropfosfamid, Bendamustin, Thymosin α-1, Aclarubicin, Fludarabin-5'-dihydrogenphosphat, Antagonisten von Purin- und Pyrimidin-Basen, Hydroxycarbamid, Aldesleukin, Pegasparase, Letrozol, Aminoglutethemid, Adriamycin, Azithromycin, Spiramycin, Cepharantin, Epothilone A und B, Mitoxanthrone, Azathioprin, Mycophenolatmofetil, c-myc-Antisense, b-myc-Antisense, Betulinsäure, Camptothecin, Camptothecin-Derivate, Melanocyte-stimulating hormon (α-MSH), aktiviertes Protein C, IL1-β-Inhibitor, Fumarsäure und deren Ester, Dermicidin, Calcipotriol, Tacalcitol, Lapachol, β-Lapachon, Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Letrozol, Goserelin, Chephalomannin, Trastuzumab, Exemestan, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren, Pentaerythrityltetranitrat, Syndnoeimine, S-Nitrosoderivate, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazol, Bacitracin, Vitronectin-Rezeptor Antagonisten, Azelastin, freie Nukleinsäuren, Nukleinsäuren in Virenüberträger inkorporiert, DNA- und RNA-Fragmente, Plasminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline, Dicloxacillin, Oxacillin, Sulfonamide, Metronidazol, Antithrombotika, Argatroban, Aspirin, Abciximab, synthetisches Antithrombin, Bivalirudin, Coumadin, Enoxoparin, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren, NF-kB oder Bcl-xL-Antisense-Oligonukleotide, Halofuginon, Nifedipin, Tocopherol Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron., natürliche und synthetisch hergestellte Steroide wie Bryophyllin A, Inotodiol, Maquirosid A, Ghalakinosid, Mansonin, Streblosid, Hydrocortison, Betamethason, Dexamethason, Fenoporfen, Ibuprofen, Indomethacin, Naproxen, Phenylbutazon, Acyclovir, Ganciclovir, Zidovudin, Antimykotika, Clotrimazol, Flucytosin, Griseofulvin, Ketoconazol, Miconazol, Nystatin, Terbinafin, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrosti-stachin, Ovatodiolide, 4,7-Oxycycloanisomelsäure, Baccharinoide B1, B2, B3 und B7, Tubeimosid, Bruceanole A, B und C, Bruceantinoside C, Yadanzioside N, und P, Isodeoxyelephantopin, Tomenphantopin A und B, Coronarin A, B, C und D, Ursolsäure, Hyptatsäure A, Zeorin, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Aristolactam-AII, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B.

11. Nanopartikel zur Verwendung nach einem der vorherigen Ansprüche, **dadurch gekennzeichnet, dass** die Nanopartikel eine Affinität zu entarteten Zellen aufweisen.

12. Nanopartikel zur Verwendung nach einem der vorherigen Ansprüche, **dadurch gekennzeichnet, dass** die Tumore ein solider Tumor sind, bevorzugt Prostatakarzinome, Gehirntumore, Sarkome, Zervix-Karzinome, Ovarialkarzinome, Mammakarzinome, Bronchialkarzinome, Melanome, Kopf-Hals Tumore, Ösophaguskarzinome, Rektumkarzinome, Pankreas-, Blasen- oder Nierenkarzinome, Metastasen der Leber, des Gehirns oder in Lymphknoten.

## Claims

1. Nanoparticles for application in a method of prophylaxis and/or therapy concerning cancerous diseases, ulcers, tumours, carcinoma or cells disturbed in proliferation, **characterised in that** the nanoparticles are provided with a positive surface charge and the nanoparticles are administered together with an anticancer drug such that the nanoparticles and the anticancer drug are present together in the degenerate cell, and that the nanoparticles and the anticancer drug are administered in separate solutions.

2. The nanoparticles for application according to Claim 1, **characterised in that** the nanoparticles are magnetic nanoparticles, preferably of a magnetic material, a ferromagnetic, anti-ferromagnetic, ferrimagnetic, anti-ferrimagnetic or superparamagnetic material.

3. The nanoparticles for application according to one of Claims 1 to 2, **characterised in that** the nanoparticles are provided with a coating of positively polarisable and/or positively ionisable substances.

4. The nanoparticles for application according to Claim 3, **characterised in that** the positively polarisable and/or positively ionisable substances contain amino groups and/or nitrogen atoms.

5. The nanoparticles for application according to Claims 3 or 4, **characterised in that** the coating consists of biologically stable or biologically inert or biostable substances, preferably of polymers, in particular of polycondensated aminosilanes, in particular of 3-aminopropyltriethoxysilane, 2-aminoethyl-330 aminopropyltrimethoxysilane, trimethoxysilylpropyldiethylentriamine or N-(6Aminohexyl)-3-aminopropyltrimethoxysilane.

6. The nanoparticles for application according to one of the preceding claims, **characterised in that** the nanoparticles consist of iron oxide, magnetite, maghemite or M(II)Fe204, where M stands for Zn, Cu, Co, Ni, Cd, Ba or Mn.

7. The nanoparticles for application according to one of the preceding claims, **characterised in that** the nanoparticles have an average diameter of 15 nm or are in the range of 1 -100 nm, preferably in the range of 10 -20 nm.

8. The nanoparticles for application according to one of the preceding claims, **characterised in that** the nanoparticles are applied in the course of hyperthermia, magnetic-liquid hyperthermia, thermotherapy with magnetic liquids, radiation therapy and/or chemotherapy.

9. The nanoparticles for application according to one of the preceding claims, **characterised in that** the anticancer drug is a cytostatic, a cytostatic agent, an anti-proliferative agent, an anti-phlogistic agent, an anti-migrative agent, an anti-angiogenic agent, an antiinflammatory agent, an anti-bacterial agent or a microtubule Inhibitor.

10. The nanoparticles for application according to one of the preceding claims, **characterised in that** the anticancer drug is selected from the group comprising: Actinomycin D, Aminoglutethimid, Amsacrin, Anastrozol, antagonists of Purin and Pyrimidin bases, Anthracycline, Aromatase inhibitor, Asparaginase, Antioestrogene, Bexaroten, Bleomycin, Buselerin, Busulfan, Camptothecin-derivate, Capecitabin, Carboplatin, Carmustin, Chlorambucil, Cisplatin, Cladribin, Cyclophosphamid, Cytarabin, Cytosinarabinosid, alkylating Cytostatics, Dacarbazin, Dactinomycin, Daunorubicin, Docetaxel, Doxorubicin (Adriamycin), Doxorubicin lipo, Epirubicin, Estramustin, Etoposid, Exemestan, Fludarabin, Fluorouracil, folic acid antagonists, Formestan, Gemcitabin, Glucocorticoide, Goselerin, hormones and hormone antagonists, Hycamtin, hydroxyurea, Idarubicin, Ifosfamid, Imatinib, Irinotecan, Letrozol, Leuprorelin, Lomustin, Melphalan, Mercaptopurin, Methotrexat, Miltefosin, Mitomycine, Mitose inhibitors, Mitoxantron, Nimustine, Oxaliplatin, Paclitaxel, Pentostatin, Procarbazin, Tamoxifen, Temozolomid, Teniposid, Testolacton, Thiotepa, Thioguanin, Topoisomerase inhibitors, Topotecan, Treosulfan, Tretinoin, Triptorelin, Trofosfamide, Vinblastin, Vincristin, Vindesin, Vinorelbin, cytostatically active antibiotics, Actinomycin D, Aminoglutethimid, Anthracycline, aromatase inhibitors, Antioestrogene, Buselerin, folic acid antagonists, Goselerin, hormone antagonists, Hycamtin, hydroxyurea, Letrozol, Mitose inhibitors, Tamoxifen, Testolacton, Sirolimus (Rapamycin), Everolimus, Pimecrolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, 4Hydroxyoxycyclophosphamid, Tropfosfamid, Bendamustin, Thymosin 0-1, Aclarubicin, Fludarabin-5'-dihydrogenphosphate, antagonists of purin and pyrimidin bases, Hydroxycarbamid, AIdesleukin, Pegasparase, Letrozol, Aminoglutethemid, Adriamycin, Azithromycin, Spiramycin, Cepharantin, Epothilone A and B, Mitoxanthrone, Azathioprin, Mycophenolatmofetil, c-myc-Antisense, b-myc-Antisense, betulinic acid, Camptothecin, camptothecin derivates, melanocyte-stimulating hormone (α-MSH), activated protein C, IL1-β inhibitor, fumaric acid and its ester, Dermicidin, Calcipotriol, Tacalcitol, Lapachol, β-Lapachon, Podophyllotoxin, Betulin, podophyllic acid-2-ethylhydrazide, Molgramostim (rhuGM-CSF), Peginterferon α-2b, Lanograstim (r-HuG-CSF), Filgrastim, Macrogol, Dacarbazin, Letrozol, Goserelin, Chephalomannin, Trastuzumab, Exemestan, Basiliximab, Daclizumab, Selectin (Cytokin antagonist), CETP inhibitor, Cadherine, Cytokin inhibitors, COX-2 inhibitors, NFkB, Angiopeptin, Ciprofloxacin, Camptothecin, Fluroblastin, bFGF antagonists, Probucol, Prostaglandine, I, II-Dimethoxycanthin-6-on, I-Hydroxy-IIMethoxycanthin-6-on, Scopolectin, Colchicin, NO donors, Pentaerythrityltetranitrate, Syndnoeimine, S-Nitrosoderivate, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin, Verapamil, Tyrosin-Kinase inhibitors (Tyrphostine), Cyclosporin A, Paclitaxel and its derivated such as 6- α -Hydroxy-Paclitaxel, Baccatin, Taxotere, Mofebutazon, Acemetacin, Diclofenac, Lonazolac, Dapson, o-Carbamoylphenoxy acetic acid, Lidocain, Ketoprofen, mefenamic acid, Piroxicam, Meloxicam, Chloroquin phosphate, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-2485I (Calbiochem), Colcemid, Cytochalasin A-E, Indanocine, Nocadazol, Bacitracin, Vitronectin receptor antagonists, Azelastin, free nucleic acids, nucleic acids incorporated in virus carriers, DNA and RNA fragments, Plasminogen activator inhibitor 1, Plasminogen activator Inhibitor 2, antisense Oligonucleotide, VEGF inhibitors, IGF-I, active agents from the group of antibiotics such as Cefadroxil, Cefazolin, Cefaclor, Cefotixin, Tobramycin, Gentamycin, Penicillin, Dicloxacillin, Oxacillin, Sulfonamide, Metronidazol, Antithrombotics, Argatroban, Aspirin, Abciximab, synthetic Antithrombin, Bivalirudin, Coumadin, Enoxoparin, GpIIb/IIIa-platelet membrane receptor, factor Xₐ-Inhibitor antibodies, Heparin, Hirudin, r-Hirudin, PPACK, Protamin, Prourokinase, Streptokinase, Warfarin, Urokinase, vasodilators, Dipyramidol, Trapidil, Nitroprusside, PDGF antagonists, Triazolopyrimidin, Seramin, ACE inhibitors, Captopril, Cilazapril, Lisinopril, Enalapril, Losartan, Thioprotease inhibitors, Prostacyclin, Vapiprost, Interferon α ,β, and γ Histamin antagonists, serotonin blockers, apoptose inhibitors, apoptose regulators, NF-kB or Bcl-xL-antisense Oligonucleotides, Halofuginon, Nifedipin, Tocopherol Tranirast, Molsidomin, Teepolyphenols, Epicatechin gallates, Epigallocatechin gallates, boswellic acids and their derivates, Leflunomid, Anakinra, Etanercept, Sulfasalazin, Etoposid, Dicloxacyllin, Tetracyclin, Triamcinolon, Mutamycin, Procainimid, retinolic acid, Quinidin, Disopyrimid, Flecainid, Propafenon, Sotolol, Amidoron, natural and synthetically produced steroids such as Bryophyllin A, Inotodiol, Maquirosid A, Ghalakinosid, Mansonin, Streblosid, Hydrocortison, Betamethason, Dexamethason, Fenoporfen, Ibuprofen, Indomethacin, Naproxen, Phenylbutazon, Acyclovir, Ganciclovir, Zidovudin, Antimykotika, Clotrimazol, Flucytosin, Griseofulvin, Ketoconazol, Miconazol, Nystatin, Terbinafin, Chloroquin, Mefloquin, Quinin, natural Terpenoids, 15 Hippocaesculin, Barringtogenol-C21 angelate, 14-Dehydroagrostistachin, Agroskerin, Agrostistachin, 17-Hydroxyagrosti-stachin, Ovatodiolide, 4,7-Oxycycloanisomelic acid, Baccharinoids BI, B2, B3 und B7, Tubeimosid, Bruceanole A, Bund C, Bruceantinoside C, Yadanzioside N, and P, Isodeoxyelephantopin, Tomenphantopin A und B, Coronarin A, B, C und D, ursolic acid, hyptatic acid A, Zeorin, Iso-Iridogerrnanal, Maytenfoliol, Effusantin A, Excisanin A and B, Longikaurin B, Sculponeatin C, Kamebaunin, Leukamenin A and B, 13, 18-dehydro-6-alpha-senecioyloxychaparrin, Taxamairin A and B, Regenilol, Triptolid, Cymarin, Apocymarin, aristolochic acid, Anopterin, Hydroxyanopterin, Anemonin, Protoanemonin, Berberin, Cheliburinchlorid, Cictoxin, Sinococulin, Bombrestatin A and B, Cudraisoflavon A, Curcumin, Dihydronitidin, Nitidinchlorid, 12-beta-Hydroxypregnadien-3,20-dion, Bilobol, Ginkgol, ginkgolic acid, Helenalin, Indicin, Indicin-N-oxid, Lasiocarpin, Inotodiol, Glykosid 1a, Podophyllotoxin, Justicidin A and B, Larreatin, Malloterin, Mallotochromanol, Isobutyrylmallotochromanol, Maquirosid A, Marchantin A, 30 Maytansin, Lycoridicin, Margetin, Pancratistatin, Liriodenin, Bispsrthenolidin, Oxoushinsunin, Aristolactam-AII, Bisparthenolidin, Periplocosid A, Ghalakinosid, ursolic acid, Deoxypsorosperrnin, Psycorubin, Ricin A, Sanguinarin, manwuweizic acid, Methylsorbifolin, Sphatheliachromen, Stizophyllin, Mansonin, Streblosid, Akagerin, Dihydrousambaraensin, Hydroxyusambarin, Strychnopentamin, Strychnophyllin,
Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A and B.

11. The nanoparticles for application according to one of the preceding claims, **characterised in that** the nanoparticles show an affinity for degenerate cells.

12. The nanoparticles for application according to one of the preceding claims, **characterised in that** the tumours are solid tumours, preferably prostate carcinoma, brain tumours, sarcomas, cervical carcinoma, ovarial carcinoma, mammary carcinoma, bronchial carcinoma, melanoma, head and neck tumour, esophagial carcinoma, rectal carcinoma, pancreatic, bladder or kidney carcinoma, metastases of the liver, of the brain or in lymph nodes.

## Revendications

1. Nanoparticules pour l'utilisation dans un procédé pour la prophylaxie et/ou le traitement des maladies cancéreuses, des ulcères, des tumeurs, des carcinomes ou dans la prolifération de cellules anormales, les nanoparticules présentant une charge de surface positive, les nanoparticules étant administrées conjointement avec un agent anti-cancéreux de telle sorte que les nanoparticules et l'agent anti-cancéreux soient présents simultanément sur la cellule dégénérée, et les nanoparticules et l'agent anti-cancéreux étant administrés dans des solutions séparées.

2. Nanoparticules pour l'utilisation selon la revendication 1, les nanoparticules étant **caractérisées en ce qu'**elles sont des nanoparticules magnétiques, de préférence en un matériau magnétique, un matériau ferromagnétique, anti-ferromagnétique, ferrimagnétique, anti-ferrimagnétique ou superparamagnétique.

3. Nanoparticules pour l'utilisation selon l'une des revendications 1 et 2, les nanoparticules présentant un revêtement en matières polarisables positivement et/ou ionisables positivement.

4. Nanoparticules pour l'utilisation selon la revendication 3, dans lesquelles les substances polarisables positivement et/ou ionisables positivement présentent des groupes amino et/ou azote.

5. Nanoparticules pour l'utilisation selon la revendication 3 ou 4, dans lesquelles le revêtement se compose de substances biologiquement stables et/ou biologiquement inertes et/ou biostables, de préférence de polymères, en particulier d'aminosilanes polycondensés, en particulier de 3-aminopropyltriéthoxysilane, 2-aminoéthyl-3-aminopropyltriméthoxysilane, triméthoxysilylpropyldiéthylènetriamine ou N-(6-aminohexyl)-3-aminopropyltriméthoxysilane.

6. Nanoparticules pour l'utilisation selon l'une des revendications précédentes, les nanoparticules se composant d'oxyde de fer, de magnétite, de maghémite ou de M(II)Fe₂O₄, où M représente Zn, Cu, Co, Ni, Cd, Ba ou Mn.

7. Nanoparticules pour l'utilisation selon l'une des revendications précédentes, les nanoparticules présentant un diamètre moyen de 15 nm ou étant situées dans une plage de tailles de 1 à 100 nm, de préférence dans la plage de 10 à 20 nm.

8. Nanoparticules pour l'utilisation selon l'une des revendications précédentes, les nanoparticules étant mises en oeuvre dans le cadre d'une hyperthermie, hyperthermie à fluide magnétique, thermothérapie avec des fluides magnétiques, radiothérapie et/ou chimiothérapie.

9. Nanoparticules pour l'utilisation selon l'une des revendications précédentes, dans lesquelles l'agent anti-cancéreux est un agent cytostatique, une substance active cytotoxique, une substance active antiproliférative, une substance active antiphlogistique, une substance active anti-migratoire, une substance active anti-angiogène, une substance active anti-inflammatoire, une substance active antibactérienne ou un inhibiteur de microtubules.

10. Nanoparticules pour l'utilisation selon l'une des revendications précédentes, l'agent anti-cancéreux étant choisi dans le groupe comprenant : l'actinomycine D, l'aminoglutéthimide, l'amsacrine, l'anastrozol, les antagonistes des bases purine et pyrimidine, les anthracyclines, les inhibiteurs d'aromatase, l'asparaginase, les anti-oestrogènes, le béxarotène, la bléomycine, la busélérine, le busulfan, les dérivés de camptothécine, la capécitabine, le carboplatine, la carmustine, le chlorambucil, le cisplatine, la cladribine, le cyclophosphamide, la cytarabine, le cytosine-arabinoside, les agents cytostatiques alkylants, la dacarbazine, la dactinomycine, la daunorubicine, le docétaxel, la doxorubicine (adriamycine), la doxorubicine lipo, l'épirubicine, l'estramustine, l'étoposide, l'exémestane, la fludarabine, le fluorouracile, les antagonistes de l'acide folique, le formestan, la gemcitabine, les glucocorticoïdes, la gosélérine, les hormones et les antagonistes d'hormones, l'hycamtin, l'hydroxyurée, l'idarubicine, l'ifosfamide, l'imatinib, l'irinotécan, le létrozol, la leuproréline, la lomustine, le melphalan, la mercaptopurine, le méthotrexate, la miltéfosine, la mitomycine, les substances antimitotiques, la mitoxantrone, la nimustine, l'oxaliplatine, le paclitaxel, la pentostatine, la procarbazine, le tamoxifène, le témozolomide, le téniposide, la testolactone, le thiotépa, la thioguanine, les inhibiteurs de topoisomérase, le topotécan, le tréosulfan, la trétinoïne, la triptoréline, le trofosfamide, la vinblastine, la vincristine, la vindésine, la vinorelbine, les antibiotiques à action cytostatique, l'actinomycine D, l'aminoglutéthimide, les anthracyclines, les inhibiteurs d'aromatase, les anti-oestrogènes, la busélérine, les antagonistes de l'acide folique, la gosélérine, les antagonistes hormonaux, l'hycamtin, l'hydroxyurée, le létrozol, les substances antimitotiques, le tamoxifène, la testolactone, le sirolimus (rapamycine), l'évérolimus, le pimécrolimus, la somatostatine, le tacrolimus, la roxithromycine, la dunaimycine, l'ascomycine, la bafilomycine, l'érythromycine, la midécamycine, la josamycine, la concanamycine, la clarithromycine, la troléandomycine, la folimycine, la cérivastatine, la simvastatine, la lovastatine, la fluvastatine, la rosuvastatine, l'atorvastatine, la pravastatine, la pitavastatine, le 4-hydroxyoxycyclophosphamide, le tropfosfamide, la bendamustine, la thymosine α-1, l'aclarubicine, le 5'-dihydrogénophosphate de fludarabine, les antagonistes des bases purine et pyrimidine, l'hydroxycarbamide, l'aldesleukine, la pégasparase, le létrozol, l'aminoglutéthimide, l'adriamycine, l'azithromycine, la spiramycine, la cépharantine, l'épothilone A et B, la mitoxanthrone, l'azathioprine, le mycophénolate mofétil, le c-myc anti-sens, le b-myc anti-sens, l'acide bétulinique, la camptothécine, les dérivés de camptothécine, l'hormone stimulant les mélanocytes (α-MSH), la protéine C activée, l'inhibiteur d'IL1-β, l'acide fumarique et ses esters, la dermicidine, le calcipotriol, le tacalcitol, le lapachol, la β-lapachone, la podophyllotoxine, la bétuline, le 2-éthylhydrazide d'acide podophyllique, le molgramostim (rhuGM-CSF), le Peg-interféron α-2b, le lanograstim (r-HuG-CSF), la filgrastim, le macrogol, la dacarbazine, le létrozol, la goséréline, la céphalomannine, le trastuzumab, l'exémestane, le basiliximab, le daclizumab, la sélectine (antagoniste de cytokines), l'inhibiteur de CETP, les cadhérines, les inhibiteurs de cytokines, l'inhibiteur de COX-2, le NFkB, l'angiopeptine, la ciprofloxacine, la camptothécine, la fluroblastine, les antagonistes de bFGF, le probucol, la prostaglandine, la 1,11-diméthoxycanthine-6-one, la 1-hydroxy-11-méthoxycanthine-6-one, la scopolectine, la colchicine, les donneurs de NO, le tétranitrate de pentaérythrityle, la syndnonimine, les dérivés S-nitroso, le tamoxifène, la staurosporine, le β-estradiol, l'α-estradiol, l'estriol, l'estron, l'éthinylestradiol, le fosfestrol, la médroxyprogestérone, l'estradiolcypionate, l'estradiolbenzoate, le tranilast, la kamébakaurine, le vérapamil, les inhibiteurs de tyrosine-kinase (tyrphostine), la cyclosporine A, le paclitaxel et ses dérivés comme le 6-α-hydroxy-paclitaxel, la baccatine, le taxotère, la mofébutazone, l'acémétacine, le diclofénac, le lonazolac, la dapsone, l'acide o-carbamoylphénoxy-acétique, la lidocaïne, le kétoprofène, l'acide méfènaminique, le piroxicam, le méloxicam, le phosphate de chloroquine, la pénicillamine, l'hydroxychloroquine, l'auranofine, l'aurothiomalate de sodium, l'oxacéprol, le célécoxib, la ß-sitostérine, l'adémétionine, la myrtécaïne, le polidocanol, le nonivamide, le lévomenthol, la benzocaïne, l'aescine, l'ellipticine, le D-24851 (Calbiochem), le colcémide, la cytochalasine A-E, l'indanocine, le nocadazol, la bacitracine, les antagonistes du récepteur de la vitronectine, l'azélastine, les acides nucléiques libres, les acides nucléiques incorporés dans des supports de transfert de virus, les fragments d'ADN et d'ARN, l'inhibiteur de l'activateur du plasminogène-1, l'inhibiteur de l'activateur du plasminogène-2, les oligonucléotides anti-sens, les inhibiteurs de VEGF, l'IGF-1, les substances actives provenant du groupe des antibiotiques tels que le céfadroxil, la céfazoline, le céfaclor, la céfotixine, la tobramycine, la gentamycine, la pénicilline, la dicloxacilline, l'oxacilline, les sulfonamides, le métronidazol, les antithrombotiques, l'argatrobane, l'aspirine, l'abciximab, l'antithrombine synthétique, la bivalirudine, la coumadine, l'énoxoparine, le récepteur membranaire des plaquettes GpIIb/IIIa, les anticorps de l'inhibiteur du facteur Xₐ, l'héparine, l'hirudine, la r-hirudine, PPACK, la protamine, la prourokinase, la streptokinase, la warfarine, l'urokinase, les vasodilatateurs, le dipyramidol, le trapidil, les nitroprussides, les antagonistes de PDGF, la triazolo-pyrimidine, la séramine, les inhibiteurs d'ACE, le captopril, le cilazapril, le lisinopril, l'énalapril, le losartan, les inhibiteurs de thioprotéase, la prostacycline, le vapiprost, l'interféron α, β et γ, les antagonistes de l'histamine, les inhibiteurs de sérotonine, les inhibiteurs d'apoptose, les régulateurs d'apoptose, les oligonucléotides anti-sens NF-kB ou Bcl-xL, l'halofuginone, la nifédipine, le tocophérol, le tranirast, la molsidomine, les polyphénols du thé, le gallate d'épicatéchine, le gallate d'épigallocatéchine, les acides boswelliques et leurs dérivés, le léflunomide, l'anakinra, l'étanercept, la sulfasalazine, l'étoposide, la dicloxacylline, la tétracycline, la triamcinolone, la mutamycine, le procaïnamide, l'acide rétinolique, la quinidine, le disopyrimide, le flécaïnide, la propafénone, le sotolol, l'amidoron, les stéroïdes d'origine naturelle ou synthétique comme la bryophylline A, l'inotodiol, le maquiroside A, le ghalakinoside, la mansonine, le strébloside, l'hydrocortisone, la bétaméthasone, la déxaméthasone, le fénoprofène, l'ibuprofène, l'indo-méthacine, le naproxène, la phénylbutazone, l'acyclovir, le ganciclovir, la zidovudine, les antimycosiques, le clotrimazol, la flucytosine, la griséofulvine, le kétoconazol, le miconazol, la nystatine, la terbinafine, la chloroquine, la méfloquine, la quinine, les terpénoïdes naturels, l'hippocaesculine, le barringtogénol-C21-angélate, la 14-déhydroagrostistachine, l'agroskérine, l'agrostistachine, la 17-hydroxyagrosti-stachine, les ovatodiolides, l'acide 4,7-oxycycloanisomélique, les baccharinoïdes B1, B2, B3 et B7, le tubéimoside, les brucéanoles A, B et C, le brucéantinoside C, les yadanziosides N et P, l'isodésoxyéléphantopine, la tomenphantopine A et B, la coronarine A, B, C et D, l'acide ursolique, l'acide hyptatique A, la zéorine, l'iso-iridogermanal, le maytenfoliol, l'effusantine A, l'excisanine A et B, la longikaurine B, la sculponéatine C, la kamébaunine, la leukaménine A et B, la 13,18-déshydro-6-alpha-sénécioyloxychaparrine, la taxamairine A et B, le régénilol, le triptolide, la cymarine, l'apocymarine, l'acide aristolochique, l'anoptérine, l'hydroxyanoptérine, l'anémonine, la protoanémonine, la berbérine, le chlorure de chéliburine, la cictoxine, la sinococuline, la bombrestatine A et B, la cudraisoflavone A, la curcumine, la dihydronitidine, le chlorure de nitidine, la 12-bêta-hydroxyprégnadiène-3, 20-dione, le bilobol, le ginkgol, l'acide ginkgolique, l'hélénaline, l'indicine, le N-oxyde d'indicine, la lasiocarpine, l'inotodiol, le glycoside 1a, la podophyllotoxine, la justicidine A et B, la larréatine, la mallotérine, le mallotochromanol, l'isobutyrylmallotochromanol, le maquiroside A, la marchantine A, la maytansine, la lycoridicine, la margétine, la pancratistatine, la liriodénine, la bisparthénolidine, l'oxoushinsunine, l'aristolactame-AII, la bisparthénolidine, le périplocoside A, les ghalakinosides, l'acide ursolique, la désoxypsorospermine, la psycorubine, la ricine A, la sanguinarine, l'acide de blé Manwu, la méthylsorbifoline, le sphathéliachromène, la stizophylline, la mansonine, le strébloside, l'akagérine, la dihydrousambaraensine, l'hydroxyusambarine, la strychnopentamine, la strychnophylline, l'usambarine, l'usambarensine, la berbérine, la liriodénine, l'oxoushinsunine, la daphnorétine, le laricirésinol, le méthoxylaricirésinol, le syringarésinol, l'umbelliféron, l'afromoson, l'acétyl-vismion B, le désacétylvismion A, le vismion A et B.

11. Nanoparticules pour l'utilisation selon l'une des revendications précédentes, les nanoparticules étant **caractérisées en ce qu'**elles présentent une affinité pour les cellules dégénérées.

12. Nanoparticules pour l'utilisation selon l'une des revendications précédentes, **caractérisées en ce que** les tumeurs sont des tumeurs solides, de préférence des carcinomes de la prostate, des tumeurs du cerveau, des sarcomes, des carcinomes du col de l'utérus, des carcinomes des ovaires, des carcinomes mammaires, des carcinomes bronchiques, des mélanomes, des tumeurs de la tête et du cou, des carcinomes de l'oesophage, des carcinomes du rectum, des carcinomes du pancréas, de la vessie ou des reins, des métastases du foie, du cerveau ou des noeuds lymphatiques.
